(19) Europäisches Patentamt
European Patent Office
Office européen des brevets

(11) Veröffentlichungsnummer: **0 585 756 A2**

# (12) EUROPÄISCHE PATENTANMELDUNG

(21) Anmeldenummer: **93113340.9**

(22) Anmeldetag: **20.08.93**

(51) Int. Cl.5: **C07D 285/13**, C07D 285/08, A01N 43/82

(30) Priorität: **02.09.92 DE 4229193**

(43) Veröffentlichungstag der Anmeldung:
**09.03.94 Patentblatt 94/10**

(84) Benannte Vertragsstaaten:
**BE CH DE ES FR GB IT LI NL**

(71) Anmelder: **BAYER AG**

**D-51368 Leverkusen(DE)**

(72) Erfinder: **Förster, Heinz, Dr.**
**Am Eckbusch 47**
**D-42113 Wuppertal(DE)**
Erfinder: **Lürssen, Klaus, Dr.**
**August-Kierspel-Strasse 145**
**D-51469 Bergisch Gladbach(DE)**
Erfinder: **Santel, Hans-Joachim, Dr.**
**Grünstrasse 9a**
**D-51371 Leverkusen(DE)**
Erfinder: **Schmidt, Robert R., Dr.**
**Im Waldwinkel 110**
**D-51467 Bergisch Gladbach(DE)**

## (54) Heteroaryloxyacetamide als Herbizide.

(57) Die Erfindung betrifft neue Heteroaryloxyacetamide der allgemeinen Formel (I),

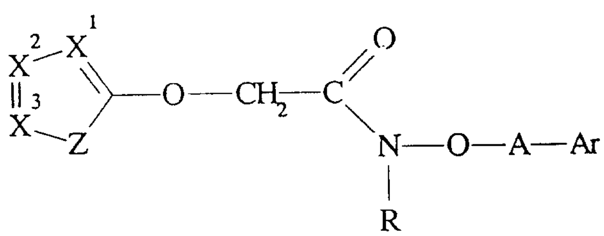

(I)

in welcher

| | |
|---|---|
| R | für Wasserstoff oder für jeweils gegebenenfalls substituiertes Alkyl, Alkenyl oder Alkinyl steht, |
| A | für einen zweifach verknüpften Alkylenrest steht, |
| Ar | für gegebenenfalls substituiertes Aryl steht, |
| $X^1$, $X^2$ und $X^3$ | unabhängig voneinander jeweils für ein Stickstoffatom oder für einen Rest C-$R^1$ stehen und |
| Z | für Sauerstoff oder Schwefel steht, wobei |
| $R^1$ | für Wasserstoff, Halogen, Cyano, für jeweils gegebenenfalls substituiertes Alkyl, Alkoxy, Alkylthio, Alkylsulfinyl oder Alkylsulfonyl oder für gegebenenfalls substituiertes Aryl steht. |

wobei jedoch mindestens einer der Reste $X^1$, $X^2$ oder $X^3$ für einen Rest C-$R^1$ steht, ein Verfahren zu ihrer Herstellung und ihre Verwendung als Herbizide.

EP 0 585 756 A2

Die Erfindung betrifft neue Heteroaryloxyacetamide, ein Verfahren zu ihrer Herstellung und ihre Verwendung als Herbizide.

Es ist bekannt, daß bestimmte Heteroaryloxyacetamide wie beispielsweise die Verbindung 2-(6-Chlor-2-benzoxazolyloxy)-N-isopropyl-N-benzyloxy-acetamid herbizide Eigenschaften besitzen (vergl. z.B. Int. Appl. PCT WO 9106544; vergl.ferner EP-A-29171 = US-Patent 4,408,055). Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden nun neue Heteroaryloxyacetamide der allgemeinen Formel (I),

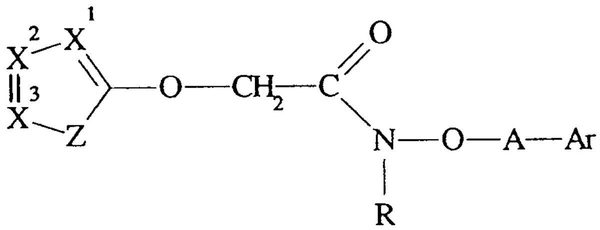

(I)

in welcher

| | |
|---|---|
| R | für Wasserstoff oder für jeweils gegebenenfalls substituiertes Alkyl, Alkenyl oder Alkinyl steht, |
| A | für einen zweifach verknüpften Alkylenrest steht, |
| Ar | für gegebenenfalls substituiertes Aryl steht, |
| $X^1$, $X^2$ und $X^3$ | unabhängig voneinander jeweils für ein Stickstoffatom oder für einen Rest C-$R^1$ stehen und |
| Z | für Sauerstoff oder Schwefel steht, wobei |
| $R^1$ | für Wasserstoff, Halogen, Cyano, für jeweils gegebenenfalls substituiertes Alkyl, Alkoxy, Alkylthio, Alkylsulfinyl oder Alkylsulfonyl oder für gegebenenfalls substituiertes Aryl steht, |

wobei jedoch mindestens einer der Reste $X^1$, $X^2$ oder $X^3$ für einen Rest C-$R^1$ steht, gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Heteroaryloxyacetamide der allgemeinen Formel (I),

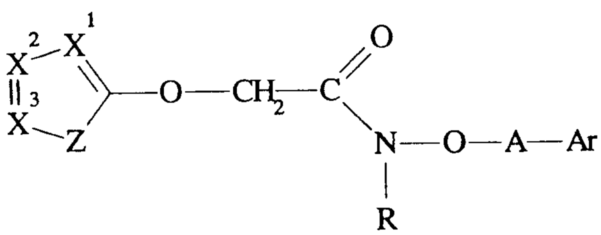

(I)

in welcher

| | |
|---|---|
| R | für Wasserstoff oder für jeweils gegebenenfalls substituiertes Alkyl, Alkenyl oder Alkinyl steht, |
| A | für einen zweifach verknüpften Alkylenrest steht, |
| Ar | für gegebenenfalls substituiertes Aryl steht, |
| $X^1$, $X^2$ und $X^3$ | unabhängig voneinander jeweils für ein Stickstoffatom oder für einen Rest C-$R^1$ stehen und |
| Z | für Sauerstoff oder Schwefel steht, wobei |
| $R^1$ | für Wasserstoff, Halogen, Cyano, für jeweils gegebenenfalls substituiertes Alkyl, Alkoxy, Alkylthio, Alkylsulfinyl oder Alkylsulfonyl oder für gegebenenfalls substituiertes Aryl steht, |

wobei jedoch mindestens einer der Reste $X^1$, $X^2$ oder $X^3$ für einen Rest C-$R^1$ steht,

erhält, wenn man substituierte Heterocyclen der Formel (II),

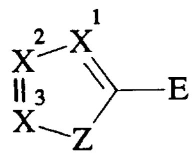

(II)

in welcher

$X^1$, $X^2$, $X^3$ und Z die oben angegebenen Bedeutungen haben und

E für eine elektronenanziehende Abgangsgruppe steht,

mit Hydroxyacetamiden der Formel (III),

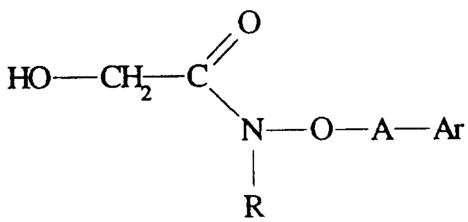

(III)

in welcher

R, A und Ar die oben angegebenen Bedeutungen haben,

gegebenenfalls in Gegenwart eines Verdünnungsmittels, gegebenenfalls in Gegenwart eines Reaktionshilfsmittels und gegebenenfalls in Gegenwart eines Katalysators umsetzt.

Schließlich wurde gefunden, daß die neuen Heteroaryloxyacetamide der allgemeinen Formel (I) herbizide Eigenschaften besitzen.

Überraschenderweise zeigen die erfindungsgemäßen Heteroaryloxyacetamide der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten Heteroaryloxyacetamide, wie beispielsweise die Verbindung 2-(6-Chlor-2-benzoxazolyloxy)-N-isopropyl-N-benzyloxy-acetamid oder die Verbindung 2-(2-Benzthiazolyloxy)-N-methyl-N-phenylacetamid, welche chemisch und wirkungsmäßig naheliegende Verbindungen sind.

Die erfindungsgemäßen Heteroaryloxyacetamide sind durch die Formel (I) allgemein definiert. Bevorzugt sind Verbindungen der Formel (I), bei welchen

R für Wasserstoff, für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen, Cyano, geradkettiges oder verzweigtes Alkoxy mit 1 bis 6 Kohlenstoffatomen, geradkettiges oder verzweigtes Alkylthio mit 1 bis 6 Kohlenstoffatomen oder Phenyl;
außerdem für gegebenenfalls einfach oder mehrfach, gleich oder verschieden durch Halogen substituiertes, geradkettiges oder verzweigtes Alkenyl mit 2 bis 8 Kohlenstoffatomen steht und außerdem für gegebenenfalls einfach oder mehrfach, gleich oder verschieden durch Halogen substituiertes, geradkettiges oder verzweigtes Alkinyl mit 2 bis 8 Kohlenstoffatomen steht

A für einen zweifach verknüpften, geradkettigen oder verzweigten Alkylenrest mit 1 bis 6 Kohlenstoffatomen steht,

Ar für einfach oder mehrfach, gleich oder verschieden substituiertes Aryl mit 6 bis 10 Kohlenstoffatomen steht, wobei als Substituenten infrage kommen:
Halogen, Cyano, Nitro, jeweils geradkettiges oder verzweigtes Alkyl, Alkoxy oder Alkylthio mit jeweils 1 bis 6 Kohlenstoffatomen oder jeweils geradkettiges oder verzweigtes Halogenalkyl, Halogenalkoxy, Halogenalkylthio mit jeweils 1 bis 6 Kohlenstoffatomen und 1 bis 13 gleichen oder verschiedenen Halogenatomen,

$X^1$, $X^2$ und $X^3$ unabhängig voneinander jeweils für ein Stickstoffatom oder für einen Rest C-$R^1$ stehen und

Z für Sauerstoff oder Schwefel steht, wobei

$R^1$ für Wasserstoff, Halogen, Cyano, für jeweils geradkettiges oder verzweigtes Alkyl, Alkoxy, Alkylthio, Alkylsulfinyl oder Alkylsulfonyl mit jeweils 1 bis 6 Kohlenstoffatomen, für jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen oder für einen Rest der Formel

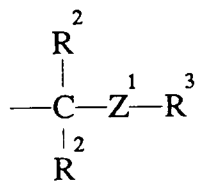

steht, wobei

$R^2$ für Wasserstoff oder Halogen steht,

$R^3$ für jeweils geradkettiges oder verzweigtes Alkyl mit 1 bis 6 Kohlenstoffatomen, Halogenalkyl mit 1 bis 6 Kohlenstoffatomen und 1 bis 13 gleichen oder verschiedenen Halogenatomen, Alkoxyalkyl mit 1 bis 6 Kohlenstoffatomen in den einzelnen Alkylteilen oder Alkylthioalkyl mit 1 bis 6 Kohlenstoffatomen in den einzelnen Alkylteilen oder für gegebenenfalls einfach oder mehrfach, gleich oder verschieden substituiertes Aryl mit 6 bis 10 Kohlenstoffatomen steht, wobei als Substituenten infrage kommen:
Halogen, Cyano, Nitro, jeweils geradkettiges oder verzweigtes Alkyl, Alkoxy oder Alkylthio mit jeweils 1 bis 6 Kohlenstoffatomen oder jeweils geradkettiges oder verzweigtes Halogenalkyl, Halogenalkoxy, Halogenalkylthio mit jeweils 1 bis 6 Kohlenstoffatomen und 1 bis 13 gleichen oder verschiedenen Halogenatomen und

$Z^1$ für Sauerstoff, Schwefel, eine Sulfinylgruppe oder eine Sulfonylgruppe steht,
wobei jedoch mindestens einer der Reste $X^1$, $X^2$ oder $X^3$ für einen Rest C-$R^1$ steht.

Besonders bevorzugt sind Verbindungen der Formel (I), bei welchen

R für Wasserstoff, für gegebenenfalls einfach substituiertes, geradkettiges oder verzweigtes Alkyl mit 1 bis 6 Kohlenstoffatomen steht, wobei als Substituenten infrage kommen: Cyano, geradkettiges oder verzweigtes Alkoxy mit 1 bis 4 Kohlenstoffatomen, geradkettiges oder verzweigtes Alkylthio mit 1 bis 4 Kohlenstoffatomen oder Phenyl;
oder für geradkettiges oder verzweigtes Halogenalkyl mit 1 bis 4 Kohlenstoffatomen und 1 bis 9 gleichen oder verschiedenen Halogenatomen steht,
außerdem für gegebenenfalls einfach bis dreifach, gleich oder verschieden durch Halogen substituiertes, geradkettiges oder verzweigtes Alkenyl mit 2 bis 6 Kohlenstoffatomen steht und
außerdem für gegebenenfalls einfach bis dreifach, gleich oder verschieden durch Halogen substituiertes, geradkettiges oder verzweigtes Alkinyl mit 2 bis 6 Kohlenstoffatomen steht und

A für einen zweifach verknüpften, geradkettigen oder verzweigten Alkylenrest mit 1 bis 4 Kohlenstoffatomen steht,

Ar für einfach bis dreifach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,

$X^1$, $X^2$ und $X^3$ unabhängig voneinander jeweils für ein Stickstoffatom oder für einen Rest C-$R^1$ stehen und

Z für Sauerstoff oder Schwefel steht, wobei

$R^1$ für Wasserstoff, Halogen, Cyano, für jeweils geradkettiges oder verzweigtes Alkyl, Alkoxy, Alkylthio, Alkylsulfinyl oder Alkylsulfonyl mit jeweils 1 bis 4 Kohlenstoffatomen, für jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen oder für einen Rest der

Formel

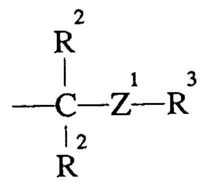

steht, wobei

$R^2$ für Wasserstoff oder Halogen steht,

$R^3$ für jeweils geradkettiges oder verzweigtes Alkyl mit 1 bis 4 Kohlenstoffatomen, Halogenalkyl mit 1 bis 4 Kohlenstoffatomen und 1 bis 9 gleichen oder verschiedenen Halogenatomen, Alkoxyalkyl mit 1 bis 4 Kohlenstoffatomen in den einzelnen Alkylteilen oder Alkylthioalkyl mit 1 bis 4 Kohlenstoffatomen in den einzelnen Alkylteilen oder für gegebenenfalls einfach bis dreifach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen und

$Z^1$ für Sauerstoff, Schwefel, eine Sulfinylgruppe oder eine Sulfonylgruppe steht,
wobei jedoch mindestens einer der Reste $X^1$, $X^2$ oder $X^3$ für einen Rest C-$R^1$ steht.

Ganz besonders bevorzugt sind Verbindungen der Formel (I), bei welchen

R für Wasserstoff, für gegebenenfalls einfach substituiertes, geradkettiges oder verzweigtes Alkyl mit 1 bis 4 Kohlenstoffatomen steht, wobei als Substituenten infrage kommen: Cyano, geradkettiges oder verzweigtes Alkoxy mit 1 bis 3 Kohlenstoffatomen, geradkettiges oder verzweigtes Alkylthio mit 1 bis 3 Kohlenstoffatomen oder Phenyl;
oder für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom - steht,
außerdem für gegebenenfalls einfach oder zweifach, gleich oder verschieden durch Halogen - insbesondere Fluor, Chlor und/oder Brom - substituiertes, geradkettiges oder verzweigtes Alkenyl mit 2 bis 4 Kohlenstoffatomen steht und
außerdem für gegebenenfalls einfach oder zweifach, gleich oder verschieden durch Halogen - insbesondere Fluor, Chlor und/oder Brom - substituiertes, geradkettiges oder verzweigtes Alkinyl mit 2 bis 4 Kohlenstoffatomen steht und

A für einen zweifach verknüpften, geradkettigen oder verzweigten Alkylenrest mit 1 bis 3 Kohlenstoffatomen steht,

Ar für einfach oder zweifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Difluormethyl, Trifluormethoxy, Difluormethoxy, Trifluormethylthio oder Difluormethylthio,

$X^1$, $X^2$ und $X^3$ unabhängig voneinander jeweils für ein Stickstoffatom oder für einen Rest C-$R^1$ stehen und

Z für Sauerstoff oder Schwefel steht, wobei

$R^1$ für Wasserstoff, Halogen - insbesondere Fluor, Chlor und/oder Brom - , Cyano, für jeweils geradkettiges oder verzweigtes Alkyl, Alkoxy, Alkylthio, Alkylsulfinyl oder Alkylsulfonyl mit jeweils 1 bis 3 Kohlenstoffatomen, für jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen - insbesondere Fluor, Chlor und/oder Brom - oder für einen Rest der Formel

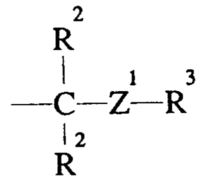

steht, wobei

$R^2$ für Wasserstoff oder Fluor oder Chlor steht,

$R^3$ für jeweils geradkettiges oder verzweigtes Alkyl mit 1 bis 3 Kohlenstoffatomen, Halogenalkyl mit 1 bis 3 Kohlenstoffatomen und 1 bis 7 gleichen oder verschiedenen Halogenatomen - insbesondere Fluor, Chlor und/oder Brom - , Alkoxyalkyl mit 1 bis 3 Kohlenstoffatomen in den einzelnen Alkylteilen oder Alkylthioalkyl mit 1 bis 3 Kohlenstoffatomen in den einzelnen Alkylteilen oder für gegebenenfalls einfach oder zweifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Difluormethyl, Trifluormethoxy, Difluormethoxy, Trifluormethylthio oder Difluormethylthio und

$Z^1$ für Sauerstoff, Schwefel, eine Sulfinylgruppe oder eine Sulfonylgruppe steht,

wobei jedoch mindestens einer der Reste $X^1$, $X^2$ oder $X^3$ für einen Rest $C\text{-}R^1$ steht.

Im einzelnen seien außer den bei den Herstellungsbeispielen genannten Verbindungen die folgenden Heteroaryloxyacetamide der allgemeinen Formel (I) genannt:

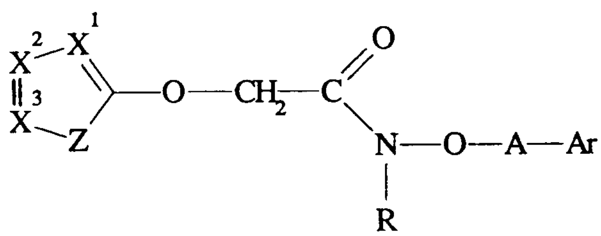

(I)

Tabelle 1:

| 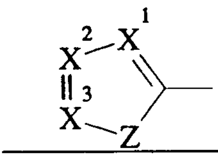 | R | A | Ar |
|---|---|---|---|
| 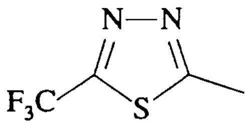 | $CH_3$ | $-CH_2-$ | $C_6H_5$ |
| 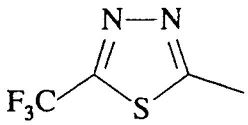 | $C_2H_5$ | $-CH_2-$ | $C_6H_5$ |
| 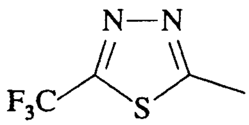 | $n-C_3H_7$ | $-CH_2-$ | $C_6H_5$ |
| 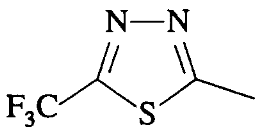 | $i-C_3H_7$ | $-CH_2-$ | $C_6H_5$ |
| 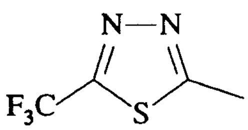 | $-CH_2-CH=CH_2$ | $-CH_2-$ | $C_6H_5$ |

Tabelle 1 (Fortsetzung):

| 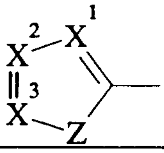 | R | A | Ar |
|---|---|---|---|
| 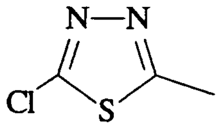 | $CH_3$ | $-CH_2-$ | $C_6H_5$ |
| 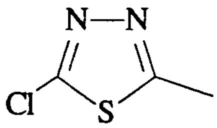 | $C_2H_5$ | $-CH_2-$ | $C_6H_5$ |
| 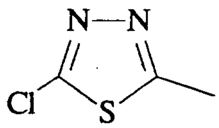 | n-$C_3H_7$ | $-CH_2-$ | $C_6H_5$ |
| 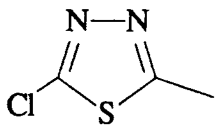 | i-$C_3H_7$ | $-CH_2-$ | $C_6H_5$ |
| 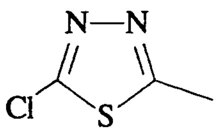 | $-CH_2-CH=CH_2$ | $-CH_2-$ | $C_6H_5$ |
| 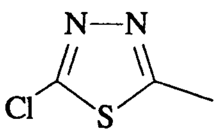 | $-CH_2-C\equiv CH$ | $-CH_2-$ | $C_6H_5$ |
| 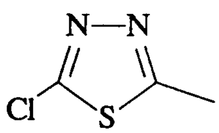 | $CH_3$ | 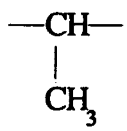 | $C_6H_5$ |
| 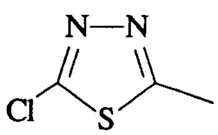 | $CH_3$ | 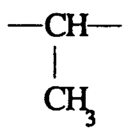 | 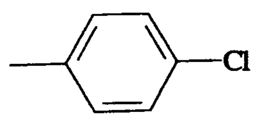 |
| 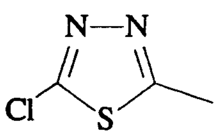 | $-CH_2-CH_2-O-CH_3$ | $-CH_2-$ | $C_6H_5$ |
| 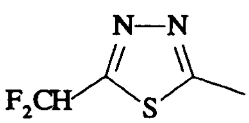 | $CH_3$ | $-CH_2-$ | $C_6H_5$ |

Tabelle 1 (Fortsetzung):

| 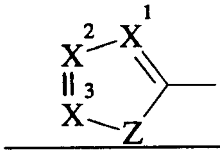 | R | A | Ar |
|---|---|---|---|
| 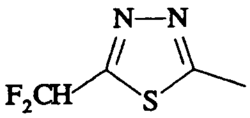 | n-$C_3H_7$ | -$CH_2$- | $C_6H_5$ |
| 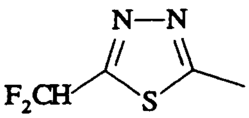 | i-$C_3H_7$ | 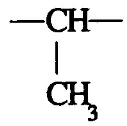 | $C_6H_5$ |
| 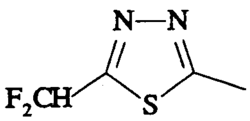 | -$CH_2$-$CH_2$-CN | -$CH_2$- | 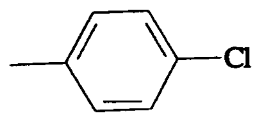 |
| 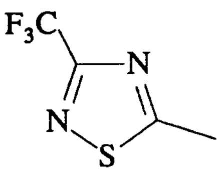 | $CH_3$ | -$CH_2$- | $C_6H_5$ |
| 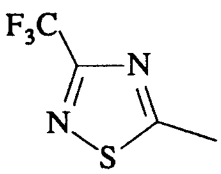 | $C_2H_5$ | -$CH_2$- | $C_6H_5$ |
| 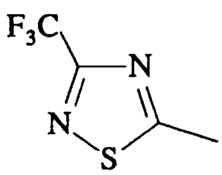 | n-$C_3H_7$ | -$CH_2$- | $C_6H_5$ |
| 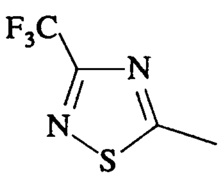 | i-$C_3H_7$ | -$CH_2$- | $C_6H_5$ |
| 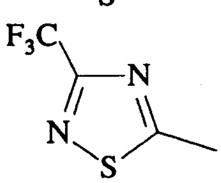 | i-$C_3H_7$ | -$CH_2$- | $C_6H_5$ |

Tabelle 1 (Fortsetzung):

| 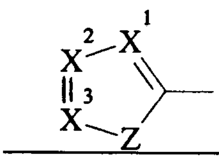 | R | A | Ar |
|---|---|---|---|
| 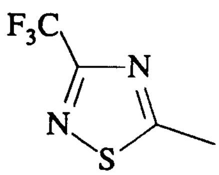 | i-$C_3H_7$ | -$CH_2$- | 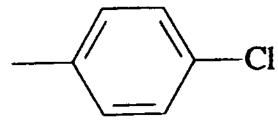 |
| 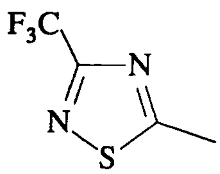 | i-$C_3H_7$ | -$CH_2$- | 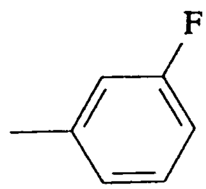 |
| 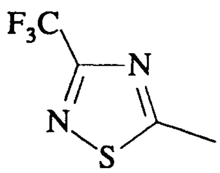 | i-$C_3H_7$ | -$CH_2$- | 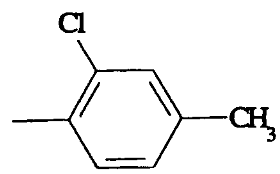 |
| 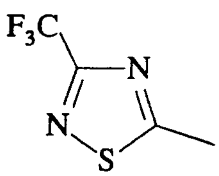 | i-$C_3H_7$ | -$CH_2$- | 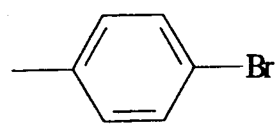 |
| 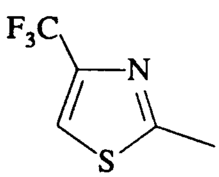 | i-$C_3H_7$ | -$CH_2$- | $C_6H_5$ |
| 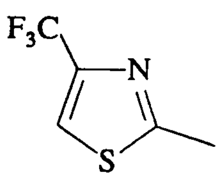 | $CH_3$ | -$CH_2$- | $C_6H_5$ |
| 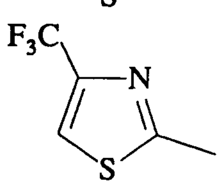 | i-$C_3H_7$ | -$CH_2$- | 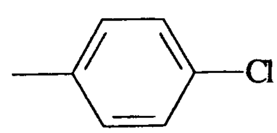 |

Tabelle1 (Fortsetzung):

| 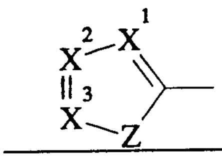 | R | A | Ar |
|---|---|---|---|
| 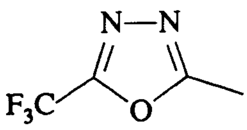 | i-$C_3H_7$ | $-CH_2-$ | $C_6H_5$ |
| 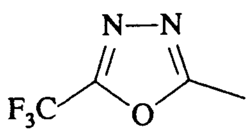 | $CH_3$ | $-CH_2-$ | $C_6H_5$ |
| 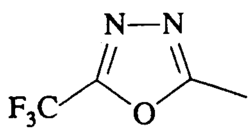 | i-$C_3H_7$ | $-CH_2-$ | 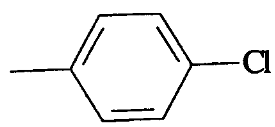 |
| 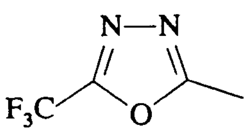 | i-$C_3H_7$ | 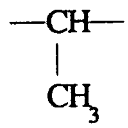 | $C_6H_5$ |
| 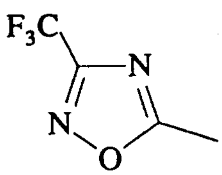 | $CH_3$ | $-CH_2-$ | $C_6H_5$ |
| 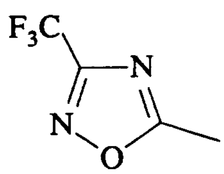 | i-$C_3H_7$ | $-CH_2-$ | $C_6H_5$ |

Verwendet man beispielsweise 2-Methylsulfonyl-5-trifluormethyl-1,3,4-thiadiazol und 2-Hydroxyessigsäure-N-methyl-N-benzyloxyamid als Ausgangsstoffe, so läßt sich der Reaktionsablauf des erfindungsgemäßen Verfahrens durch das folgende Formelschema darstellen:

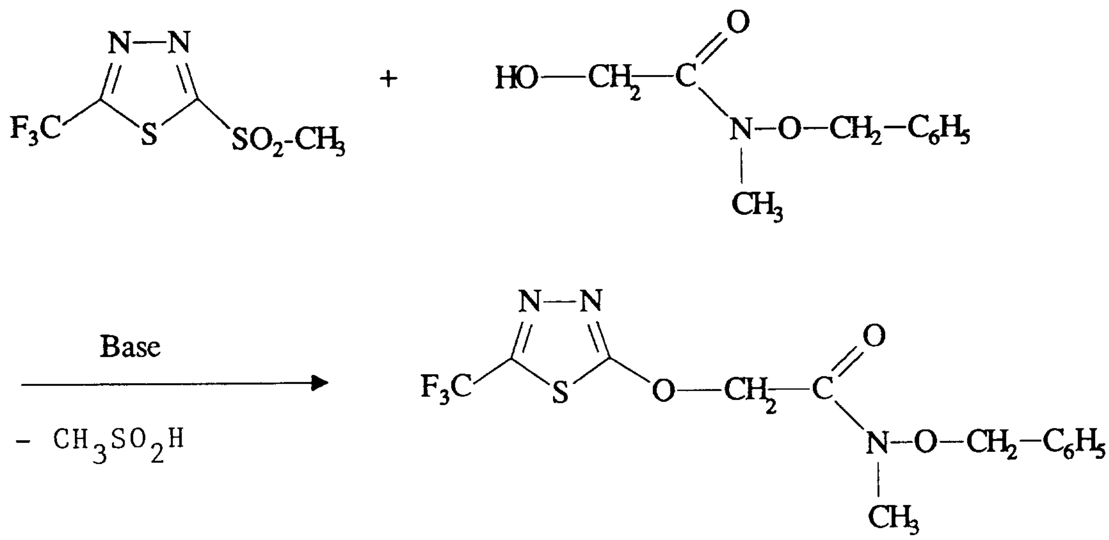


Die zur Durchführung des erfindungsgemäßen Verfahrens als Ausgangsverbindung benötigten substituierten Heterocyclen sind durch die Formel (II) allgemein definiert. In dieser Formel (II) stehen $X^1$, $X^2$, $X^3$ und Z vorzugsweise für diejenigen Reste, die bereits im Zusammenhang mit der Beschreibung der erfindungsgemäßen Stoffe der Formel (I) als bevorzugt für diese Substituenten genannt wurden.

E steht für einen üblichen Abgangsrest, vorzugsweise für Halogen, insbesondere für Chlor, Brom oder Iod oder für jeweils gegebenenfalls substituiertes Alkylsulfonyloxy oder Arylsulfonyloxy, wie insbesondere Methansulfonyloxy, Trifluormethansulfonyloxy oder p-Toluolsulfonyloxy.

Die substituierten Heterocyclen der Formel (II) sind bekannt oder erhältlich in Analogie zu bekannten Verfahren (vergl. z.B. EP 348 737).

Die zur Durchführung des erfindungsgemäßen Verfahrens weiterhin als Edukte erforderlichen Hydroxyacetamide sind durch die Formel (III) allgemein definiert. In dieser Formel (III) stehen R, A und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die Hydroxyacetamide der Formel (III) sind bekannt oder erhältlich in Analogie zu bekannten Verfahren (vergl. z.B. Int. Appl. PCT WO 9106544).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole; wie Methanol, Ethanol, n- oder i-Propanol, deren Gemische mit Wasser, reines Wasser oder wässrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt sind wässrige Natriumchloridlösungen.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines geeigneten

Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}$/$C_{15}$-alkylammoniumchlorid, Trimethyl-$C_{13}$/$C_{15}$-alkylammoniumbromid, Dibenzyl-dimethyl-ammoniummethylsulfat, Dimethyl-C12/C14-alkyl-benzylammoniumchlorid, Dimethyl-$C_{12}$/$C_{14}$-alkylbenzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung des erfindungsgemäßen Verfahrens in einem größeren Bereich variiert werden. Im allgemeinen arbeitet man bei Temperaturen zwischen -50°C und +110°C, vorzugsweise bei Temperaturen zwischen -20°C und +80°C.

Zur Durchführung des erfindungsgemäßen Verfahrens setzt man pro Mol substituiertem Heterocyclus der Formel (II) im allgemeinen 0,5 bis 5,0 Mol, vorzugsweise 0,8 bis 1,5 Mol Hydroxyacetamid der Formel (III), 0,5 bis 5,0 Mol, vorzugsweise 0,8 bis 1,5 Mol Base als Reaktionshilfsmittel und gegebenenfalls 0,001 bis 2,0 Mol, vorzugsweise 0,001 bis 1,0 Mol Phasentransferkatalysator ein.

Die Reaktionsdurchführung, Aufarbeitung und Isolierung der Reaktionsprodukte erfolgt nach bekannten Verfahren (vergl. hierzu beispielsweise EP 348 737 bzw. Int. Appl. PCT WO 9106544 oder die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-` Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von monokotylen Unkräutern in dikotylen Kulturen wie beispielsweise Soja einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel könne z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe,

wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle,Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon` Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfüron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

Herstellungsbeispiele:

Beipiel 1:

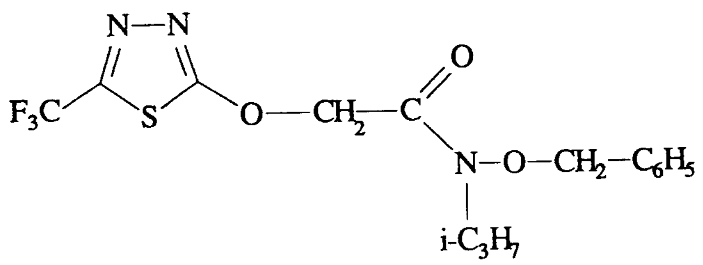

Eine Suspension von 7,1 g (0,025 Mol) Hydroxyessigsäure-N-isopropyl-N-benzyloxyamid, 5,8 g (0,025 Mol) 2-Methylsulfonyl-5-trifluormethyl-1,3,4-thiadiazol, 3,7 g (0,027 mol) Kaliumcarbonat und 0,5 g Tetrabutylammoniumbromid in 50 ml Aceton wird bei bei 0 °C bis 5 °C 12 Stunden gerührt. Zur Aufarbeitung gibt man Wasser zur Reaktionsmischung, extrahiert mit Methyl-t-butyl-ether, wäscht die organische Phase mit Wasser, trocknet über Natriumsulfat und entfernt das Lösungsmittel im Vakuum..

Man erhält 9,0 g (96 % der Theorie) 2-[(5-Trifluormethyl-1,3,4-thiadiazol-2-yl)-oxy]-N-isopropyl-N-benzyloxy-acetamid als Öl mit Brechungsindex $n_D^{20}$ = 1,4945.

In entsprechender Weise erhält man die folgenden Heteroaryloxyacetamide der allgemeinen Formel (I):

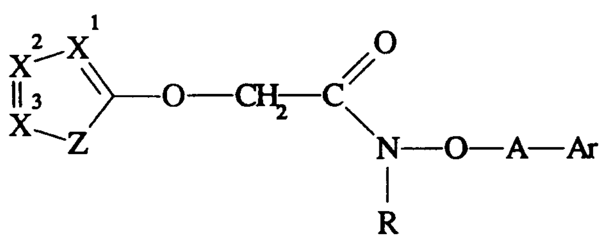

(I)

Tabelle 2:

| Bsp.-Nr. | 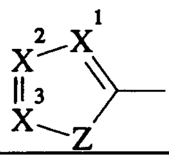 | R | A | Ar | physikalische Eigenschaften |
|---|---|---|---|---|---|
| 2 | 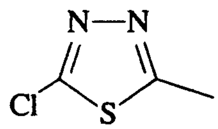 | i-$C_3H_7$ | $-CH_2-$ | $C_6H_5$ | $n_D^{20} = 1{,}5331$ |
| 3 | 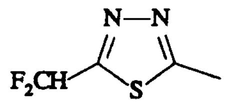 | i-$C_3H_7$ | $-CH_2-$ | $C_6H_5$ | $n_D^{20} = 1{,}5171$ |
| 4 | 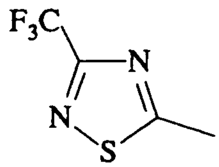 | i-$C_3H_7$ | $-CH_2-$ | $C_6H_5$ | $n_D^{20} = 1{,}4958$ |

Anwendungsbeispiele:

In den folgenden Anwendungsbeispielen wurde die nachstehend aufgeführte Verbindung als Vergleichssubstanz eingesetzt:

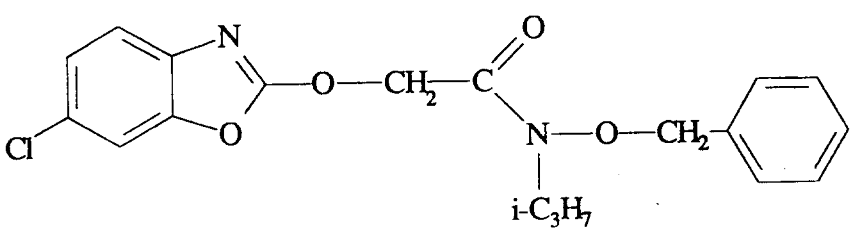

(A)

2-(6-Chlor-2-benzoxazolyloxy)-N-isopropyl-N-benzyloxy-acetamid (vergl. z.B. Int. Appl. PCT WO 9106544)

Beispiel A:

**Pre-emergence-Test / Gewächshaus**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung
Eine deutliche Überlegenheit in der Wirksamkeit bei vergleichbarer Nutzpflanzenselektivität gegenüber dem Stand der Technik zeigen in diesem Test z.B. die Verbindungen gemäß den Herstellungsbeispielen 1,2 und 4.

## Patentansprüche

**1.** Heteroaryloxyacetamide der allgemeinen Formel (I),

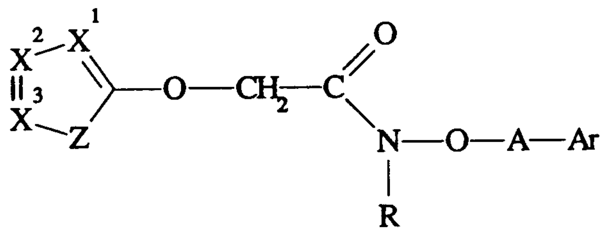

(I)

in welcher

| | |
|---|---|
| R | für Wasserstoff oder für jeweils gegebenenfalls substituiertes Alkyl, Alkenyl oder Alkinyl steht, |
| A | für einen zweifach verknüpften Alkylenrest steht, |
| Ar | für gegebenenfalls substituiertes Aryl steht, |
| $X^1$, $X^2$ und $X^3$ | unabhängig voneinander jeweils für ein Stickstoffatom oder für einen Rest C-$R^1$ stehen und |
| Z | für Sauerstoff oder Schwefel steht, wobei |
| $R^1$ | für Wasserstoff, Halogen, Cyano, für jeweils gegebenenfalls substituiertes Alkyl, Alkoxy, Alkylthio, Alkylsulfinyl oder Alkylsulfonyl oder für gegebenenfalls substituiertes Aryl steht, |

wobei jedoch mindestens einer der Reste $X^1$, $X^2$ oder $X^3$ für einen Rest C-$R^1$ steht.

**2.** Heteroaryloxyacetamide der allgemeinen Formel (I) gemäß Anspruch 1, dadurch gekennzeichnet, daß

| | |
|---|---|
| R | für Wasserstoff, für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen: Halogen, Cyano, geradkettiges oder verzweigtes Alkoxy mit 1 bis 6 Kohlenstoffatomen, geradkettiges oder verzweigtes Alkylthio mit 1 bis 6 Kohlenstoffatomen oder Phenyl; außerdem für gegebenenfalls einfach oder mehrfach, gleich oder verschieden durch Halogen substituiertes, geradkettiges oder verzweigtes Alkenyl mit 2 bis 8 Kohlenstoffatomen steht und außerdem für gegebenenfalls einfach oder mehrfach, gleich oder verschieden durch Halogen substituiertes, geradkettiges oder verzweigtes Alkinyl mit 2 bis 8 Kohlenstoffatomen steht |
| A | für einen zweifach verknüpften, geradkettigen oder verzweigten Alkylenrest mit 1 bis 6 Kohlenstoffatomen steht, |
| Ar | für einfach oder mehrfach, gleich oder verschieden substituiertes Aryl mit 6 bis 10 Kohlenstoffatomen steht, wobei als Substituenten infrage kommen: Halogen, Cyano, Nitro, jeweils geradkettiges oder verzweigtes Alkyl, Alkoxy oder Alkylthio mit jeweils 1 bis 6 Kohlenstoffatomen oder jeweils geradkettiges oder verzweigtes Halogenalkyl, Halogenalkoxy, Halogenalkylthio mit jeweils 1 bis 6 Kohlenstoffatomen und 1 bis 13 gleichen oder verschiedenen Halogenatomen, |
| $X^1$, $X^2$ und $X^3$ | unabhängig voneinander jeweils für ein Stickstoffatom oder für einen Rest C-$R^1$ stehen und |
| Z | für Sauerstoff oder Schwefel steht, wobei |
| $R^1$ | für Wasserstoff, Halogen, Cyano, für jeweils geradkettiges oder verzweigtes Alkyl, Alkoxy, Alkylthio, Alkylsulfinyl oder Alkylsulfonyl mit jeweils 1 bis 6 Kohlenstoffatomen, für jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen oder |

für einen Rest der Formel

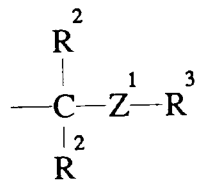

steht, wobei

$R^2$ für Wasserstoff oder Halogen steht,

$R^3$ für jeweils geradkettiges oder verzweigtes Alkyl mit 1 bis 6 Kohlenstoffatomen, Halogenalkyl mit 1 bis 6 Kohlenstoffatomen und 1 bis 13 gleichen oder verschiedenen Halogenatomen, Alkoxyalkyl mit 1 bis 6 Kohlenstoffatomen in den einzelnen Alkylteilen oder Alkylthioalkyl mit 1 bis 6 Kohlenstoffatomen in den einzelnen Alkylteilen oder für gegebenenfalls einfach oder mehrfach, gleich oder verschieden substituiertes Aryl mit 6 bis 10 Kohlenstoffatomen steht, wobei als Substituenten infrage kommen:
Halogen, Cyano, Nitro, jeweils geradkettiges oder verzweigtes Alkyl, Alkoxy oder Alkylthio mit jeweils 1 bis 6 Kohlenstoffatomen oder jeweils geradkettiges oder verzweigtes Halogenalkyl, Halogenalkoxy, Halogenalkylthio mit jeweils 1 bis 6 Kohlenstoffatomen und 1 bis 13 gleichen oder verschiedenen Halogenatomen und

$Z^1$ für Sauerstoff, Schwefel, eine Sulfinylgruppe oder eine Sulfonylgruppe steht,

wobei jedoch mindestens einer der Reste $X^1$, $X^2$ oder $X^3$ für einen Rest C-$R^1$ steht.

**3.** Heteroaryloxyacetamide der allgemeinen Formel (I) gemäß Anspruch 1, dadurch gekennzeichnet, daß

R für Wasserstoff, für gegebenenfalls einfach substituiertes, geradkettiges oder verzweigtes Alkyl mit 1 bis 6 Kohlenstoffatomen steht, wobei als Substituenten infrage kommen: Cyano, geradkettiges oder verzweigtes Alkoxy mit 1 bis 4 Kohlenstoffatomen, geradkettiges oder verzweigtes Alkylthio mit 1 bis 4 Kohlenstoffatomen oder Phenyl;
oder für geradkettiges oder verzweigtes Halogenalkyl mit 1 bis 4 Kohlenstoffatomen und 1 bis 9 gleichen oder verschiedenen Halogenatomen steht,
außerdem für gegebenenfalls einfach bis dreifach, gleich oder verschieden durch Halogen substituiertes, geradkettiges oder verzweigtes Alkenyl mit 2 bis 6 Kohlenstoffatomen steht und
außerdem für gegebenenfalls einfach bis dreifach, gleich oder verschieden durch Halogen substituiertes, geradkettiges oder verzweigtes Alkinyl mit 2 bis 6 Kohlenstoffatomen steht und

A für einen zweifach verknüpften, geradkettigen oder verzweigten Alkylenrest mit 1 bis 4 Kohlenstoffatomen steht,

Ar für einfach bis dreifach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen,

$X^1$, $X^2$ und $X^3$ unabhängig voneinander jeweils für ein Stickstoffatom oder für einen Rest C-$R^1$ stehen und

Z für Sauerstoff oder Schwefel steht, wobei

$R^1$ für Wasserstoff, Halogen, Cyano, für jeweils geradkettiges oder verzweigtes Alkyl, Alkoxy, Alkylthio, Alkylsulfinyl oder Alkylsulfonyl mit jeweils 1 bis 4 Kohlenstoffatomen, für jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen oder für einen Rest der Formel

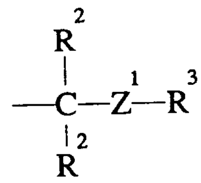

steht, wobei

$R^2$ für Wasserstoff oder Halogen steht,

$R^3$ für jeweils geradkettiges oder verzweigtes Alkyl mit 1 bis 4 Kohlenstoffatomen, Halogenalkyl mit 1 bis 4 Kohlenstoffatomen und 1 bis 9 gleichen oder verschiedenen Halogenatomen, Alkoxyalkyl mit 1 bis 4 Kohlenstoffatomen in den einzelnen Alkylteilen oder Alkylthioalkyl mit 1 bis 4 Kohlenstoffatomen in den einzelnen Alkylteilen oder für gegebenenfalls einfach bis dreifach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen und

$Z^1$ für Sauerstoff, Schwefel, eine Sulfinylgruppe oder eine Sulfonylgruppe steht,

wobei jedoch mindestens einer der Reste $X^1$, $X^2$ oder $X^3$ für einen Rest C-$R^1$ steht.

**4.** Heteroaryloxyacetamide der allgemeinen Formel (1) gemäß Anspruch 1, dadurch gekennzeichnet, daß

R für Wasserstoff, für gegebenenfalls einfach substituiertes, geradkettiges oder verzweigtes Alkyl mit 1 bis 4 Kohlenstoffatomen steht, wobei als Substituenten infrage kommen:
Cyano, geradkettiges oder verzweigtes Alkoxy mit 1 bis 3 Kohlenstoffatomen, geradkettiges oder verzweigtes Alkylthio mit 1 bis 3 Kohlenstoffatomen oder Phenyl;
oder für geradkettiges oder verzweigtes Halogenalkyl mit 1 bis 3 Kohlenstoffatomen und 1 bis 7 gleichen oder verschiedenen Halogenatomen - insbesondere Fluor, Chlor und/oder Brom - steht,
außerdem für gegebenenfalls einfach oder zweifach, gleich oder verschieden durch Halogen - insbesondere Fluor, Chlor und/oder Brom - substituiertes, geradkettiges oder verzweigtes Alkenyl mit 2 bis 4 Kohlenstoffatomen steht und
außerdem für gegebenenfalls einfach oder zweifach, gleich oder verschieden durch Halogen - insbesondere Fluor, Chlor und/oder Brom - substituiertes, geradkettiges oder verzweigtes Alkinyl mit 2 bis 4 Kohlenstoffatomen steht und

A für einen zweifach verknüpften, geradkettigen oder verzweigten Alkylenrest mit 1 bis 3 Kohlenstoffatomen steht,

Ar für einfach oder zweifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Difluormethyl, Trifluormethoxy, Difluormethoxy, Trifluormethylthio oder Difluormethylthio,

$X^1$, $X^2$ und $X^3$ unabhängig voneinander jeweils für ein Stickstoffatom oder für einen Rest C-$R^1$ stehen und

Z für Sauerstoff oder Schwefel steht, wobei

$R^1$ für Wasserstoff, Halogen - insbesondere Fluor, Chlor und/oder Brom - , Cyano, für jeweils geradkettiges oder verzweigtes Alkyl, Alkoxy, Alkylthio, Alkylsulfinyl oder Alkylsulfonyl mit jeweils 1 bis 3 Kohlenstoffatomen, für jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen - insbesondere Fluor, Chlor und/oder Brom - oder für einen Rest der Formel

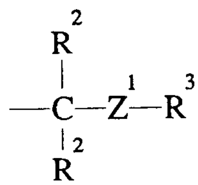

steht, wobei

| | |
|---|---|
| $R^2$ | für Wasserstoff oder Fluor oder Chlor steht, |
| $R^3$ | für jeweils geradkettiges oder verzweigtes Alkyl mit 1 bis 3 Kohlenstoffatomen, Halogenalkyl mit 1 bis 3 Kohlenstoffatomen und 1 bis 7 gleichen oder verschiedenen Halogenatomen - insbesondere Fluor, Chlor und/oder Brom - , Alkoxyalkyl mit 1 bis 3 Kohlenstoffatomen in den einzelnen Alkylteilen oder Alkylthioalkyl mit 1 bis 3 Kohlenstoffatomen in den einzelnen Alkylteilen oder für gegebenenfalls einfach oder zweifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s-oder t-Butoxy, Methylthio, Ethylthio, Trifluormethyl, Difluormethyl, Trifluormethoxy, Difluormethoxy, Trifluormethylthio oder Difluormethylthio und |
| $Z^1$ | für Sauerstoff, Schwefel, eine Sulfinylgruppe oder eine Sulfonylgruppe steht, |

wobei jedoch mindestens einer der Reste $X^1$, $X^2$ oder $X^3$ für einen Rest C-$R^1$ steht.

**5.** Verfahren zur Herstellung von Heteroaryloxyacetamiden der allgemeinen Formel (I)

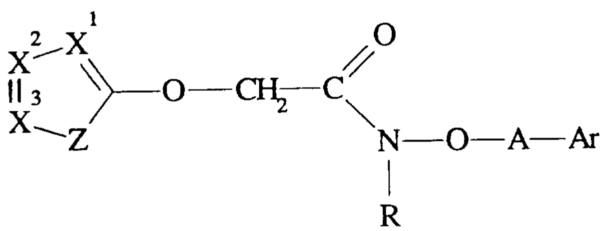

(I)

in welcher

| | |
|---|---|
| R | für Wasserstoff oder für jeweils gegebenenfalls substituiertes Alkyl, Alkenyl oder Alkinyl steht, |
| A | für einen zweifach verknüpften Alkylenrest steht, |
| Ar | für gegebenenfalls substituiertes Aryl steht, |
| $X^1$, $X^2$ und $X^3$ | unabhängig voneinander jeweils für ein Stickstoffatom oder für einen Rest C-$R^1$ stehen und |
| Z | für Sauerstoff oder Schwefel steht, wobei |
| $R^1$ | für Wasserstoff, Halogen, Cyano, für jeweils gegebenenfalls substituiertes Alkyl, Alkoxy, Alkylthio, Alkylsulfinyl oder Alkylsulfonyl oder für gegebenenfalls substituiertes Aryl steht, |

wobei jedoch mindestens einer der Reste $X^1$, $X^2$ oder $X^3$ für einen Rest C-$R^1$ steht,
dadurch gekennzeichnet, daß man substituierte Heterocyclen der Formel (II),

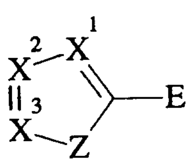

(II)

in welcher

| | |
|---|---|
| $X^1$, $X^2$, $X^3$ und Z | die oben angegebenen Bedeutungen haben und |

E für eine elektronenanziehende Abgangsgruppe steht, mit Hydroxyacetamiden der Formel (III),

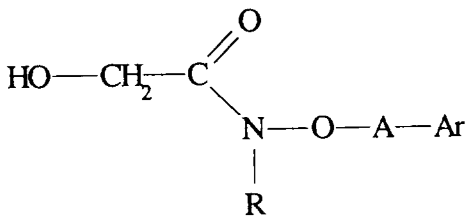

(III)

in welcher

R, A und Ar die oben angegebenen Bedeutungen haben,

gegebenenfalls in Gegenwart eines Verdünnungsmittels, gegebenenfalls in Gegenwart eines Reaktionshilfsmittels und gegebenenfalls in Gegenwart eines Katalysators umsetzt.

**6.** Herbizide Mittel, gekennzeichnet durch einen Gehalt an mindestens einem Heteroaryloxyacetamid der allgemeinen Formel (I) gemäß der Ansprüche 1 bis 5.

**7.** Verfahren zur Bekämpfung von unerwünschten Pflanzen, dadurch gekennzeichnet, daß man Heteroaryloxyacetamide der allgemeinen Formel (I) gemäß der Ansprüche 1 bis 5 auf Pflanzen und/oder ihren Lebensraum einwirken läßt.

**8.** Verwendung von Heteroaryloxyacetamiden der allgemeinen Formel (I) gemäß der Ansprüche 1 bis 5 zur Bekämpfung von unerwünschten Pflanzen.

**9.** Verfahren zur Herstellung von herbiziden Mitteln, dadurch gekennzeichnet, daß man Heteroaryloxyacetamide der allgemeinen Formel (I) gemäß der Ansprüche 1 bis 5 mit Streckmitteln und/oder oberflächenaktiven Substanzen vermischt.